(19) Europäisches Patentamt
European Patent Office
Office européen des brevets

(11) Publication number: **0 471 298 B1**

(12) **EUROPEAN PATENT SPECIFICATION**

(45) Date of publication of patent specification: **02.11.95**

(51) Int. Cl.[6]: **C07D 401/06**, A61K 31/44

(21) Application number: **91113336.1**

(22) Date of filing: **08.08.91**

(54) **Carbamoyl-1-(pyridinylalkyl)-1H-indoles, indolines and related analogs.**

(30) Priority: **13.08.90 US 566724**

(43) Date of publication of application:
**19.02.92 Bulletin 92/08**

(45) Publication of the grant of the patent:
**02.11.95 Bulletin 95/44**

(84) Designated Contracting States:
**AT BE CH DE DK ES FR GB GR IT LI LU NL SE**

(56) References cited:
**EP-A- 0 332 968**
**DE-A- 2 239 648**
**FR-A- 2 591 595**
**GB-A- 2 102 795**

**CHEMICAL ABSTRACTS, vol. 70, no. 5, February 3, 1969, Columbus, Ohio, USA SHEINKMAN, A.K. et al. "Synthesis and pharmacology of N-(pyridylalkyl)-indolines and -indoles." page 1973, column 2, abstract-no. 19 857b & Khim.-Farm. Zh. 1968, 2(9), 29-35**

(73) Proprietor: **HOECHST-ROUSSEL PHARMACEUTICALS INCORPORATED**
**Route 202-206 North**
**Somerville**
**New Jersey 08876 (US)**

(72) Inventor: **Effland, Richard Charles**
**544 Rolling Hills Road**
**Bridgewater, NJ 08807 (US)**
Inventor: **Davis, Larry**
**P.O. Box 129,**
**Bird Lane**
**Sergeantsville, NJ 08557 (US)**
Inventor: **Olsen, Gordon E.**
**8K Franklin Greens**
**Somerset, NJ 08873 (US)**

(74) Representative: **Isenbruck, Günter, Dr. et al**
**HOECHST AKTIENGESELLSCHAFT**
**Zentrale Patentabteilung**
**Gebäude F 821**
**D-65926 Frankfurt am Main (DE)**

EP 0 471 298 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to compounds of the formula

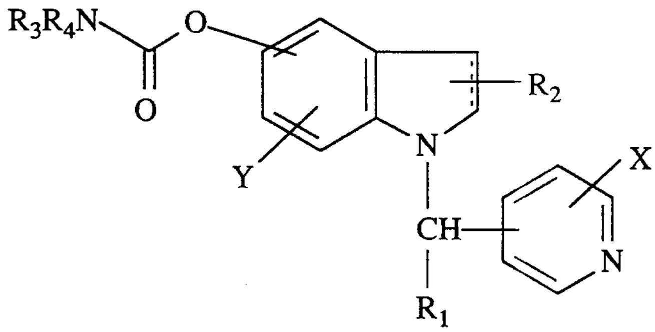

(I)

wherein

- $R_1$ is hydrogen, $(C_1\text{-}C_6)$-alkyl, phenyl-$(C_1\text{-}C_6)$-alkyl, $(C_2\text{-}C_6)$-alkenyl or $(C_2\text{-}C_6)$-alkynyl;
- $R_2$ is hydrogen, $(C_1\text{-}C_6)$-alkyl, $(C_2\text{-}C_6)$-alkenyl, formyl or cyano;
- $R_3$ is hydrogen or $(C_1\text{-}C_6)$-alkyl;
- $R_4$ is $(C_1\text{-}C_6)$-alkyl, phenyl-$(C_1\text{-}C_6)$-alkyl, $(C_3\text{-}C_8)$-cycloalkyl, phenyl, furanyl, thienyl, pyrrolyl, pyridinyl, furanyl-$(C_1\text{-}C_6)$-alkyl, thienyl-$(C_1\text{-}C_6)$-alkyl, pyrrolyl-$(C_1\text{-}C_6)$-alkyl, pyridinyl-$(C_1\text{-}C_6)$-alkyl,

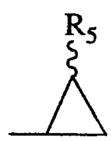 ,

or $NR_3R_4$ taken together constitute

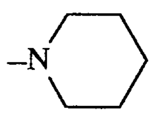 , 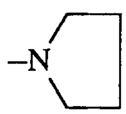 , 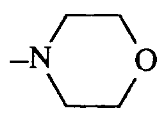 ,

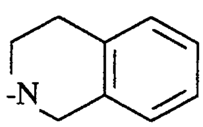 or 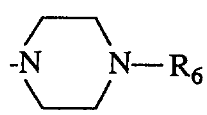 ;

- $R_5$ is hydrogen, $(C_1\text{-}C_6)$-alkyl or phenyl;
- $R_6$ is hydrogen, $(C_1\text{-}C_6)$-alkyl, phenyl, phenyl-$(C_1\text{-}C_6)$-alkyl, formyl, $(C_1\text{-}C_6)$-alkylcarbonyl or phenyl-$(C_1\text{-}C_6)$-alkylcarbonyl,
- X and Y are independently hydrogen, nitro, amino, halogen, $(C_1\text{-}C_6)$-alkyl, $(C_1\text{-}C_6)$-alkoxy or hydroxy,

wherein the phenyl radical in each occurence may be substituted with 1 or 2 substituents independently selected from $(C_1\text{-}C_6)$-alkyl, $(C_1\text{-}C_6)$-alkoxy, halogen or trifluormethyl; or the pharmaceutically acceptable acid addition salts thereof, and where applicable, the geometric and optical isomers and racemic mixtures thereof.

Preferred are compounds of formula I wherein

- $R_1$ is hydrogen, $(C_1\text{-}C_6)$-alkyl, phenyl-$(C_1\text{-}C_6)$-alkyl, $(C_2\text{-}C_6)$-alkenyl or $(C_2\text{-}C_6)$-alkynyl;
- $R_2$ is hydrogen, $(C_1\text{-}C_6)$-alkyl, $(C_2\text{-}C_6)$-alkenyl, formyl or cyano;

$R_3$ is hydrogen;
$R_4$ is $(C_1-C_6)$-alkyl, phenyl-$(C_1-C_6)$-alkyl, $(C_3-C_8)$-cycloalkyl;
X and Y are independently hydrogen, nitro, amino, halogen, $(C_1-C_6)$-alkyl, $(C_1-C_6)$-alkoxy or hydroxy,

wherein the phenyl radical in each occurence may be substituted with 1 or 2 substituents independently selected from $(C_1-C_6)$-alkyl, $(C_1-C_6)$-alkoxy, halogen or trifluormethyl; or the pharmaceutically acceptable acid addition salts thereof, and where applicable, the geometric and optical isomers and racemic mixtures thereof.

Most preferred are compounds of formula I wherein

$R_1$ is hydrogen, $(C_1-C_6)$-alkyl, phenyl-$(C_1-C_6)$-alkyl, $(C_2-C_6)$-alkenyl or $(C_2-C_6)$-alkynyl;
$R_2$ is hydrogen, $(C_1-C_6)$-alkyl, $(C_2-C_6)$-alkenyl, formyl or cyano;
$R_3$ is hydrogen;
$R_4$ is $(C_1-C_6)$-alkyl, phenyl-$(C_1-C_6)$-alkyl;
X and Y are independently hydrogen, nitro, amino, halogen, $(C_1-C_6)$-alkyl, $(C_1-C_6)$-alkoxy or hydroxy,

wherein the phenyl radical in each occurence may be substituted with 1 or 2 substituents independently selected from $(C_1-C_6)$-alkyl, $(C_1-C_6)$-alkoxy, halogen or trifluormethyl; or the pharmaceutically acceptable acid addition salts thereof, and where applicable, the geometric and optical isomers and racemic mixtures thereof.

The compounds of this invention are useful for the treatment of various memory dysfunctions characterized by a cholinergic deficit and thus may be indicated in the treatment of Alzheimer's disease.

The dotted lines present in Formula (I) signifies an optional double bond. When the double bond is present, the formula I compounds represent indoles.

Throughout the specification and appended claims, a given chemical formula or name shall encompass all geometrical and optical isomers and racemic mixtures where such isomers and mixtures exist.

The compounds of this invention are prepared in the following manner. The substituents $R_1$, $R_2$, $R_3$, $R_4$, $R_5$, $R_6$, X and Y are as defined above unless otherwise indicated.

Compound II of the formula

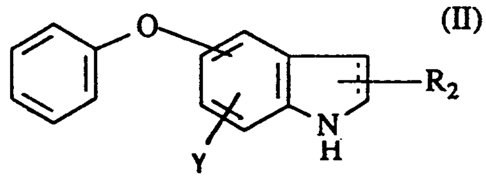

(II)

is reacted with a haloalkylpyridine hydrochloride of the formula

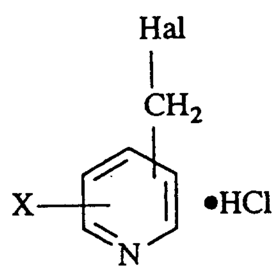

where Hal is halogen, to afford Compound III of the formula

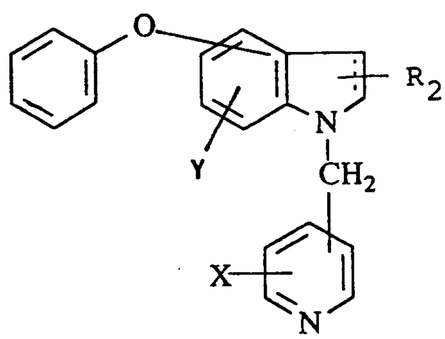

(III)

This reaction typically takes place in the presence of a base, such as potassium hydroxide and a solvent such as dimethylsulfoxide or dimethylformamide at ambient temperature for 1 to 20 hours. Compound II is commerically available or can be synthesized from known compounds according to methods known to the art.

Compound III is hydrogenated in a routine manner, for instance, using a noble metal catalyst under a hydrogen atmosphere, to afford Compound IV of the formula

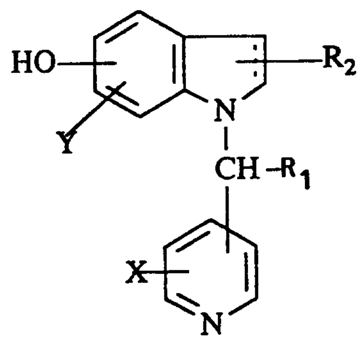

(IV)

where $R_1$ is H.

The catalyst is selected from palladium or platinum on carbon. The reaction typically takes place at a temperature of about 20 °C to 70 °C for 1 to 20 hours.

Alternatively, to prepare compounds where $R_1 \neq H$, Compound III is reacted with n-butyllithium and a halide of the formula $R_1$-Hal, where $R_1$ is as previously defined and Hal is chlorine or bromine, to afford a compound of the formula

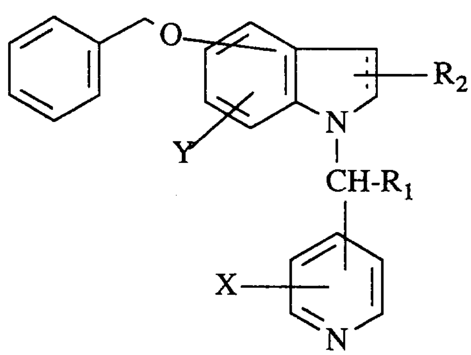

(IIIa)

Typically, this reaction is conducted in tetrahydrofuran or ether at a temperature of -80° to 0°C for 1 to 6 hours.

Compound IIIa is subsequently hydrogenated in a manner similar to that described above to afford Compound IV where $R_1 \neq$ hydrogen.

Compound IV is allowed to react with an isocyanate of the formula $R_4-N=C=O$ in the presence of a base such as potassium carbonate in a suitable solvent such as tetrahydrofuran at a temperature of about 15°C to 50°C for 1 to 50 hours to afford compound I. Alternatively, compound IV is allowed to react with carbonyldiimidazole and an amine of the formula

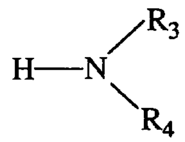

in a routine manner to afford Compound I.

The novel compounds of this invention are useful for the treatment of various memory dysfunctions characterized by a cholinergic deficit, such as that found in Alzheimer's disease.

This utility is manifested by the ability of these compounds to inhibit the enzyme acetylcholinesterase and thereby increase acetylcholine levels in the brain.

## Cholinesterase Inhibition Assay

Cholinesterases are found throughout the body, both in the brain and in serum. However, only brain acetylcholinesterease (AChE) distribution is correlated with central cholinergic innervation. This same innervation is suggested to be weakened in Alzheimer patients. We have determined in vitro inhibition of acetylcholinesterase activity in rat striatum according to the method described below.

## In Vitro Inhibition of Acetylcholinesterase Activity in Rat Striatum

Acetylcholinesterase (AChE), which is sometimes called true or specific cholinesterase, is found in nerve cells, skeletal muscle, smooth muscle, various glands and red blood cells. AChE may be distinguished from other cholinesterases by substrate and inhibitor specificities and by regional distribution. Its distribution in the brain correlates with cholinergic innervation and subfractionation shows the highest level in nerve terminals.

It is generally accepted that the physiological role of AChE is the rapid hydrolysis and inactivation of acetylcholine. Inhibitors of AChE show marked cholinomimetic effects in cholinergically-innervated effector organs and have been used therapeutically in the treatment of glaucoma, myasthenia gravis and paralytic ileus. However, recent studies have suggested that AChE inhibitors may also be beneficial in the treatment of Alzheimer's dementia.

The method described below was used in this invention for assaying anticholinesterase activity. This is a modification of the method of Ellman et al., Biochem. Pharmacol. 7, 88 (1961).

## Procedure

A. Reagents -
1. 0.05 M Phosphate buffer, pH 7.2
   (a) 6.85 g $NaH_2PO_4 \cdot H_2O$/100 mi distilled $H_2O$
   (b) 13.40 g $Na_2HPO_4 \cdot 7H_2O$/100 ml distilled $H_2O$
   (c) add (a) to (b) until pH reaches 7.2
   (d) Dilute 1:10
2. Substrate in buffer
   (a) 198 mg acetylthiocholine chloride (10 mM)
   (b) bring to 100 ml with 0.05 M phosphate buffer, pH 7.2 (reagent 1)
3. DTNB in buffer
   (a) 19.8 mg 5,5-dithiobisnitrobenzoic acid (DTNB) (0.5 mM)
   (b) bring to 100 ml with 0.05 M phosphate buffer, pH 7.2 (reagent 1)

4. A 2mM stock solution of the test drug is made up in a suitable solvent and bring to volume with 0.5 mM DTNB (reagent 3). Drugs are serially diluted (1:10) such that the final concentration (in cuvette) is $10^{-4}$ M and screened for activity. If active, $IC_{50}$ values are determined from the inhibitory activity of subsequent concentrations.

B. Tissue Preparation -

Male Wistar rats are decapitated, brains rapidly removed, corpora striata dissected free, weighed and homogenized in 19 volumes (approximately 7 mg protein/ml) of 0.05 M phosphate buffer, pH 7.2, using a Potter-Elvehjem homogenizer. A 25 microliter aliquot of the homogenate is added to 1 ml of vehicle or various concentrations of the test drug and preincubated for 10 minutes at 37 °C.

C. Assay

Enzyme activity is measured with the Beckman DU-50 spectrophotometer. This method can be used for $IC_{50}$ determinations and for measuring kinetic constants.

Instrument Settings

Kinetics Soft-Pac Module #598273 (10)
Program #6 Kindata:
Source - Vis
Wavelength - 412 nm
Sipper - none
Cuvettes - 2 ml cuvettes using auto 6-sampler
Blank - 1 for each substrate concentration
Interval time - 15 seconds (15 or 30 seconds for kinetics)
Total time - 5 minutes (5 or 10 minutes for kinetics)
Plot - yes
Span - autoscale
Slope - increasing
Results - yes (gives slope)
Factor - 1

Reagents are added to the blank and sample cuvettes as follows:

| | |
|---|---|
| Blank: | 0.8 ml Phosphate Buffer/DTNB |
| | 0.8 ml Buffer/Substrate |
| Control: | 0.8 ml Phosphate Buffer/DTNB/Enzyme |
| | 0.8 ml Phosphate Buffer/Substrate |
| Drug: | 0.8 ml Phosphate Buffer/DTNB/Drug/Enzyme |
| | 0.8 ml Phosphate Buffer/Substrate |

Blank values are determined for each run to control non-enzymatic hydrolysis of substrate and these values are automatically substracted by the kindata program available on kinetics soft-pac module. This program also calculates the rate of absorbance change for each cuvette.

For $IC_{50}$ Determinations:

Substrate concentration is 10 mM diluted 1:2 in assay yielding final concentration of 5 mM. DTNB concentration is 0.5 mM yielding 0.25 mM final concentration

$$\% \text{ Inhibition} = \frac{\text{slope control - slope drug}}{\text{slope control}} \times 100$$

$IC_{50}$ values are calculated from log-probit analysis.

Results of this assay for representative compounds of this invention and physostigmine are presented in Table 1.

Table 1

| Inhibition of Brain Acetylcholinesterase Activity | |
|---|---|
| Compound | Inhibitory Concentration $IC_{50}$ (μM) |
| 1-(4-pyridinylmethyl)-1H-indol-5-yl methylcarbamate | 6.83 |
| 1-(4-pyridinylmethyl)-1H-indol-5-yl phenylmethylcarbamate | 12.48 |
| Physostigmine (Reference compound) | 0.006 |

Effective quantities of the compounds of the present invention may be administered to a subject by any one of various methods, for example, orally as in capsules or tablets, parenterally in the form of sterile solutions or suspensions, and in some cases intravenously in the form of sterile solutions. The compounds of the present invention, while effective themselves, may be formulated and administered in the form of their pharmaceutically acceptable addition salts for purposes of stability, convenience of crystallization, increased solubility and the like.

Preferred pharmaceutically acceptable addition salts include salts of inorganic acids such as hydrochloric, hydrobromic, sulfuric, nitric, phosphoric and perchloric acids; as well as organic acids such as tartaric, citric, acetic, succinic, maleic, fumaric, and oxalic acids.

The active compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 75% of the weight of the unit. The amount of compound present in such composition is such that a suitable dosage will be obtained. Preferred compositions and preparations according to the present invention are prepared so that an oral dosage unit form contains between 1.0-300 mgs of active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel™, corn starch and the like; a lubricant such as magnesium stearate or Sterotex®; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as fatty oil. Other dosage unit forms may contain other various materials which modify the physical form of the doseage unit, for example, as coatings. Thus tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of the aforesaid compound, but may be varied between 0.5 and about 30% of the weight thereof. The amount of active compound in such compositions is such that a suitable dosage will be obtained. Preferred compositions and preparations according to the present invention are prepared so that a parenteral dosage unit contains between 0.5 to 100 mgs of active compound.

The solutions or suspensions may also include the following components; a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

Examples of the compounds of the invention are listed below:

1-(4-pyridinylmethyl)-1H-indol-5-yl methylcarbamate;
1-(4-pyridinylmethyl)-1H-indol-5-yl butylcarbamate;
1-(4-pyridinylmethyl)-1H-indol-5-yl phenylmethylcarbamate;
1-[1-(4-pyridinyl)butyl]-1H-indol-5-yl methylcarbamate;

1-[1-(4-pyridinyl)butyl]-1H-indol-5-yl phenylmethyl carbamate;
3-methyl-1-(4-pyridinylmethyl)-1H-indol-5-yl methylcarbamate;
1-[1-(3-fluoro-4-pyridinyl)butyl]-1H-indol-5-yl ethylcarbamate;
1-[1-(4-pyridinyl)butyl]-1H-indol-5-yl dimethylcarbamate;
6-fluoro-1-(4-pyridinylmethyl)-1H-indol-5-yl butylcarbamate;
1-[1-(3-fluoro-4-pyridinyl)butyl]-3-methyl-1H-indol-5-yl methylcarbamate;
2,3-dihydro-1-(4-pyridinylmethyl)-1H-indol-5-yl methylcarbamate;
2,3-dihydro-1-[1-(4-pyridinyl)butyl]-1H-indol-5-yl methylcarbamate;
2,3-dihydro-1-[1-(3-fluoro-4-pyridinyl)butyl]-1H-indol-5-yl methylcarbamate;
2,3-dihydro-1-(4-pyridinylmethyl)-1H-indol-5-yl dimethylcarbamate;
1-[1-(4-pyridinyl)butyl]-1H-indol-5-yl 2-phenylcyclopropylcarbamate;
1-[1-(4-pyridinyl)propyl]-1H-indol-5-yl cyclohexylcarbamate;
3-ethyl-1-(3-fluoro-4-pyridinylmethyl)-1H-indol-5-yl-(4-phenylmethyl-piperazin-1-carboxylate);
1-[1-(4-pyridinyl)butyl]-1H-indol-5-yl piperidin-1-carboxylate;
1-[1-(3-fluoro-4-pyridinyl)propyl]-1H-indol-5-yl morpholin-4-carboxylate;
2,3-dihydro-1-[1-(4-pyridinyl)propyl]-1H-indol-5-yl cyclohexylcarbamate;
2,3-dihydro-1-[1-(4-pyridinyl)propyl]-1H-indol-5-yl 2-phenylcyclopropylcarbamate;
2,3-dihydro-1-[1-(3-fluoro-4-pyridinyl)propyl]-1H-indol-5-yl piperidin-1-carboxylate; and
2,3-dihydro-6-fluoro-1-[1-(4-pyridinyl)butyl]-1H-indol-5-yl methylcarbamate.

The following examples are for illustrative purposes and are not to be construed as limiting the invention disclosed herein. All temperatures are given in degrees centigrade (°C) unless indicated otherwise.

## Example 1

### 1-(4-Pyridinylmethyl)-1H-indol-5-yl methylcarbamate

To a solution of 1-(4-pyridinylmethyl)-1H-indol-5-ol (2.4 g) in 50 ml tetrahydrofuran, was added milled $K_2CO_3$ (1.5 g), followed by methyl isocyanate (0.65 ml). After stirring at ambient temperature for three hours, the mixture was filtered and the filtrate evaporated to a solid, 3.0 g, m.p. 170°C. This material was eluted on a silica gel column with 2% methanol/dichloromethane via high pressure liquid chromatography (HPLC) and the desired fractions were combined, then evaporated to yield 2.4 g of 1-(4-pyridinylmethyl)-1H-indol-5-yl methylcarbamate, as a solid, m.p. 179-180°C.

| Analysis: | | | |
|---|---|---|---|
| Calculated for $C_{16}H_{15}N_3O_2$: | 68.31%C | 5.38%H | 14.94%N |
| Found: | 68.28%C | 5.47%H | 14.97%N |

## Example 2

### 1-(4-Pyridinylmethyl)-1H-indol-5-yl butylcarbamate hydrochloride

To a solution of 1-(4-pyridinylmethyl)-1H-indol-5-ol (3.0 g) in 50 ml tetrahydrofuran, was added milled $K_2CO_3$ (1.8 g) followed by butyl isocyanate (1.5 ml). After stirring at ambient temperature for four hours, the mixture was filtered, and the filtrate evaporated to an oil, (~4.5 g) which was eluted on a silica gel column with ethyl acetate/dichloromethane (1:1) via HPLC. The desired fractions were combined and evaporated to yield 3.6 g of a solid, m.p. 135-137°C. This material was dissolved in methanol, the pH adjusted to 1 with ethereal-HCl and diluted with ether. The resultant precipitate was collected and dried to give 3.0 g of 1-(4-pyridinylmethyl)-1H-indol-5-yl butylcarbamate hydrochloride, m.p. 230°C(dec).

| Analysis: | | | |
|---|---|---|---|
| Calculated for $C_{19}H_{21}N_3O_2 \cdot HCl$: | 63.41%C | 6.16%H | 11.68%N |
| Found: | 63.48%C | 6.18%H | 11.68%N |

**Example 3**

**1-(4-Pyridinylmethyl)-1H-indol-5-yl phenylmethylcarbamate**

To a solution of 1-(4-pyridinylmethyl)-1H-indol-5-ol (2.2 g) in 50 ml tetrahydrofuran, was added milled $K_2CO_3$ (1.4 g) followed by phenylmethyl isocyanate (1.2 ml). After stirring at ambient temperature for twenty hours, the mixture was filtered, and the filtrate evaporated to an oil, (~3.5 g) which was eluted on a silica gel column with ethyl acetate/dichloromethane (1:1) via HPLC. The desired fractions were combined and evaporated to yield 2.8 g of 1-(4-pyridinylmethyl)-1H-indol-5-yl phenylmethylcarbamate, m.p. 112-114 °C.

| Analysis: | | | |
|---|---|---|---|
| Calculated for $C_{22}H_{19}N_3O_2$: | 73.93%C | 5.36%H | 11.76%N |
| Found: | 73.90%C | 5.45%H | 11.68%N |

**Example 4**

**1-[1-(4-Pyridinyl)butyl]-1H-indol-5-yl methylcarbamate**

To a solution of 1-[1-(4-pyridinyl)butyl]-1H-indol-5-ol (2.4 g) in 50 ml tetrahydrofuran, was added milled $K_2CO_3$ (1.3 g) followed by methyl isocyanate (0.53 ml). After stirring at ambient temperature for three hours, the mixture was filtered, and the filtrate evaporated to an oil (3.0 g) which was eluted on a silica gel column with ethyl acetate via HPLC. The desired fraction was evaporated to yield 2.2 g of 1-[1-(4-pyridinyl)butyl]-1H-indol-5-yl methylcarbamate, as a solid, m.p. 128-9 °C.

| Analysis: | | | |
|---|---|---|---|
| Calculated for $C_{19}H_{21}N_3O_2$: | 70.56%C | 6.55%H | 12.99%N |
| Found: | 70.31%C | 6.42%H | 12.87%N |

**Example 5**

**1-[1-(4-Pyridinyl)butyl]-1H-indol-5-yl phenylmethylcarbamate**

To a solution of 1-[1-(4-pyridinyl)butyl]-1H-indol-5-ol (2.2 g) in 50 ml tetrahydrofuran, was added milled $K_2CO_3$ (1.29 g), followed by phenylmethyl isocyanate (1.0 ml). After stirring at ambient temperature for twenty hours, the mixture was filtered, and the filtrate evaporated to an oil, (3.3 g) which was eluted on a silica gel column with ethyl acetate/dichloromethane (1:1) via HPLC. The desired fraction was evaporated to yield 2.6 g of 1-[1-(4-pyridinyl)butyl]-1H-indol-5-yl phenylmethylcarbamate, as a solid, 119-21 °C.

| Analysis: | | | |
|---|---|---|---|
| Calculated for $C_{25}H_{25}N_3O_2$: | 75.16%C | 6.31%H | 10.52%N |
| Found: | 75.11%C | 6.39%H | 10.47%N |

**Claims**

**Claims for the following Contracting States : AT, BE, CH, DE, DK, FR, GB, IT, LI, LU, NL, SE**

1. A compound of the formula

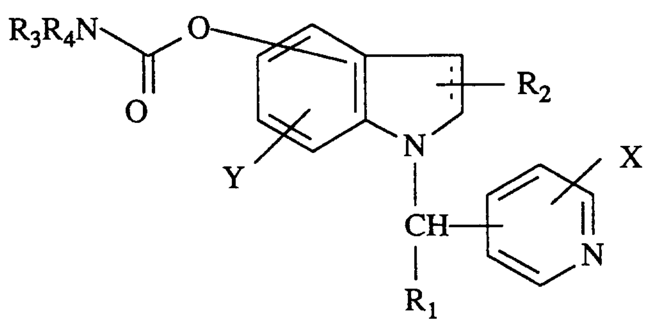

(I)

wherein

| | |
|---|---|
| $R_1$ | is hydrogen, $(C_1$-$C_6)$-alkyl, phenyl-$(C_1$-$C_6)$-alkyl, $(C_2$-$C_6)$-alkenyl or $(C_2$-$C_6)$-alkynyl; |
| $R_2$ | is hydrogen, $(C_1$-$C_6)$-alkyl, $(C_2$-$C_6)$-alkenyl, formyl or cyano; |
| $R_3$ | is hydrogen or $(C_1$-$C_6)$-alkyl; |
| $R_4$ | is $(C_1$-$C_6)$-alkyl, phenyl-$(C_1$-$C_6)$-alkyl, $(C_3$-$C_8)$-cycloalkyl, phenyl, furanyl, thienyl, pyrrolyl, pyridinyl, furanyl-$(C_1$-$C_6)$-alkyl, thienyl-$(C_1$-$C_6)$-alkyl, pyrrolyl-$(C_1$-$C_6)$-alkyl, pyridinyl-$(C_1$-$C_6)$-alkyl, |

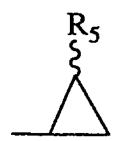

,

or $NR_3R_4$ taken together constitute

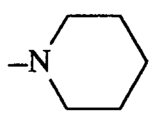

,

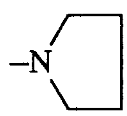

,

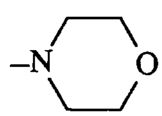

,

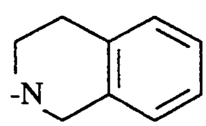

or

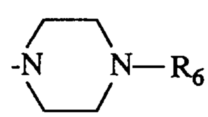

;

| | |
|---|---|
| $R_5$ | is hydrogen, $(C_1$-$C_6)$-alkyl or phenyl; |
| $R_6$ | is hydrogen, $(C_1$-$C_6)$-alkyl, phenyl, phenyl-$(C_1$-$C_6)$-alkyl, formyl, $(C_1$-$C_6)$-alkylcarbonyl or phenyl-$(C_1$-$C_6)$-alkylcarbonyl, |
| X and Y | are independently hydrogen, nitro, amino, halogen, $(C_1$-$C_6)$-alkyl, $(C_1$-$C_6)$-alkoxy or hydroxy, |

wherein the phenyl radical in each occurence may be substituted with 1 or 2 substituents independently selected from $(C_1$-$C_6)$-alkyl, $(C_1$-$C_6)$-alkoxy, halogen or trifluormethyl and wherein the dotted line signifies an optional double bond; or the pharmaceutically acceptable acid addition salts thereof, and where applicable, the geometric and optical isomers and racemic mixtures thereof.

**2.** The compound as defined in claim 1 wherein $R_3$ is hydrogen and $R_4$ is selected from $(C_1\text{-}C_6)$-alkyl, phenyl-$(C_1\text{-}C_6)$-alkyl and $(C_3\text{-}C_8)$-cycloalkyl where the phenyl radical may be substituted as indicated in claim 1.

**3.** The compound as defined in claim 2 wherein $R_3$ is hydrogen and $R_4$ $(C_1\text{-}C_6)$-alkyl.

**4.** The compound as defined in claim 2 wherein $R_3$ is hydrogen and $R_4$ is phenyl-$(C_1\text{-}C_6)$-alkyl; where the phenyl radical may be substituted as indicated in claim 1.

**5.** The compound as defined in claim 1 which is selected from the group consisting of
1-(4-pyridinylmethyl)-1H-indol-5-yl methylcarbamate,
1-(4-pyridinylmethyl)-1H-indol-5-yl butylcarbamate or its hydrochloride,
1-(4-pyridinylmethyl)-1H-indol-5-yl phenylmethylcarbamate,
1-[1-(4-pyridinyl)butyl]-1H-indol-5-yl methylcarbamate and
1-[1-(4-pyridinyl)butyl]-1H-indol-5-yl phenylmethylcarbamate.

**6.** A pharmaceutical composition comprising a compound as defined in claim 1 in an amount effective for alleviating a memory dysfunction characterized by a cholinergic deficit and a pharmaceutically acceptable carrier therefor.

**7.** A method of synthesizing a compound as defined in claim 1 wherein $R_3$ is hydrogen, which comprises reacting a compound of the formula (IV)

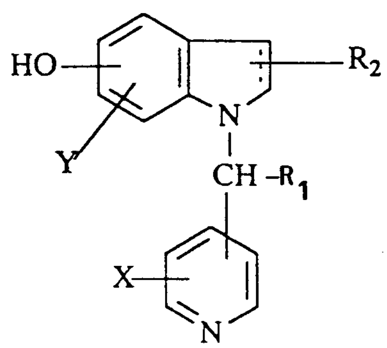

(IV)

with an isocyanate of the formula

$R_4—N=C=O$

in the presence of a base in a suitable solvent.

**8.** The method of claim 7 wherein the isocyanate is selected from methyl isocyanate, butyl isocyanate and phenylmethyl isocyanate.

**9.** A method of synthesizing a compound as defined in claim 1 which comprises reacting a compound of the formula (IV)

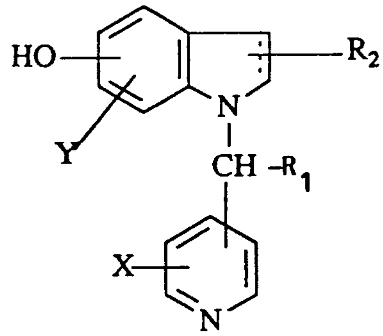

(IV)

with carbonyldiimidazole and an amine of the formula

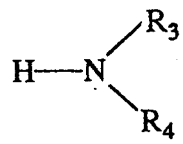

in the presence of a base in a suitable solvent.

**10.** The method of claim 9 wherein the base is potassium carbonate and the solvent is tetrahydrofuran.

**Claims for the following Contracting States : ES, GR**

**1.** A method of synthesizing a compound

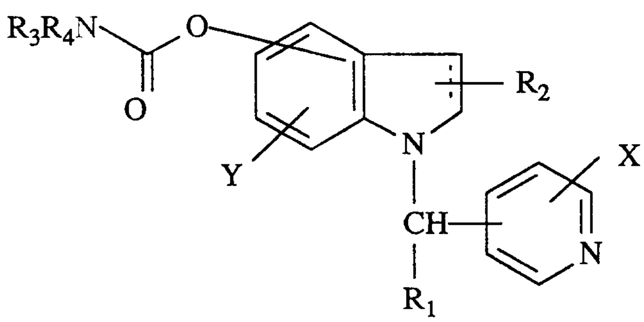

(I)

wherein

| | |
|---|---|
| $R_1$ | is hydrogen, ($C_1$-$C_6$)-alkyl, phenyl-($C_1$-$C_6$)-alkyl, ($C_2$-$C_6$)-alkenyl or ($C_2$-$C_6$)-alkynyl; |
| $R_2$ | is hydrogen, ($C_1$-$C_6$)-alkyl, ($C_2$-$C_6$)-alkenyl, formyl or cyano; |
| $R_3$ | is hydrogen or ($C_1$-$C_6$)-alkyl; |
| $R_4$ | is ($C_1$-$C_6$)-alkyl, phenyl-($C_1$-$C_6$)-alkyl, ($C_3$-$C_8$)-cycloalkyl, phenyl, furanyl, thienyl, pyrrolyl, pyridinyl, furanyl-($C_1$-$C_6$)-alkyl, thienyl-($C_1$-$C_6$)-alkyl, pyrrolyl-($C_1$-$C_6$)-alkyl, pyridinyl-($C_1$-$C_6$)-alkyl, |

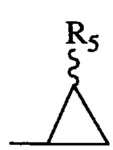

,

or $NR_3R_4$ taken together constitute

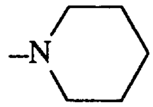 , 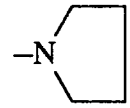 , 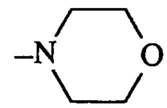 ,

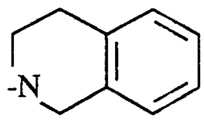 or 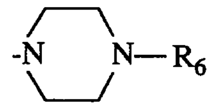 ;

- $R_5$ is hydrogen, $(C_1-C_6)$-alkyl or phenyl;
- $R_6$ is hydrogen, $(C_1-C_6)$-alkyl, phenyl, phenyl-$(C_1-C_6)$-alkyl, formyl, $(C_1-C_6)$-alkylcarbonyl or phenyl-$(C_1-C_6)$-alkylcarbonyl,
- X and Y are independently hydrogen, nitro, amino, halogen, $(C_1-C_6)$-alkyl, $(C_1-C_6)$-alkoxy or hydroxy,

wherein the phenyl radical in each occurence may be substituted with 1 or 2 substituents independently selected from $(C_1-C_6)$-alkyl, $(C_1-C_6)$-alkoxy, halogen or trifluormethyl and wherein the dotted line signifies an optional double bond; or the pharmaceutically acceptable acid addition salts thereof, and where applicable, the geometric and optical isomers and racemic mixtures thereof which comprises
reacting a compound of the formula (IV)

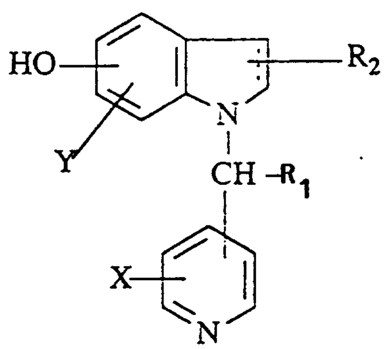 (IV)

with carbonyldiimidazole and an amine of the formula

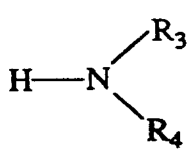

in the presence of a base in a suitable solvent.

2. The method of claim 1 wherein the base is potassium carbonate and the solvent is tetrahydrofuran.

3. A method of synthesizing a compound as defined in claim 1 wherein $R_3$ is hydrogen, which comprises reacting a compound of the formula (IV)

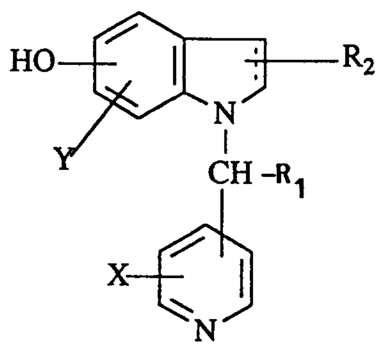

(IV)

with an isocyanate of the formula

$R_4—N=C=O$

in the presence of a base in a suitable solvent.

4. The method of claim 3 wherein the isocyanate is selected from methyl isocyanate, butyl isocyanate and phenylmethyl isocyanate.

5. The method as defined in claim 1 or 3 wherein a compound is produced which is selected from the group consisting of
1-(4-pyridinylmethyl)-1H-indol-5-yl methylcarbamate,
1-(4-pyridinylmethyl)-1H-indol-5-yl butylcarbamate or its hydrochloride,
1-(4-pyridinylmethyl)-1H-indol-5-yl phenylmethylcarbamate,
1-[1-(4-pyridinyl)butyl]-1H-indol-5-yl methylcarbamate and
1-[1-(4-pyridinyl)butyl]-1H-indol-5-yl phenylmethylcarbamate.

**Patentansprüche**
**Patentansprüche für folgende Vertragsstaaten : AT, BE, CH, DE, DK, FR, GB, IT, LI, LU, NL, SE**

1. Verbindung der Formel

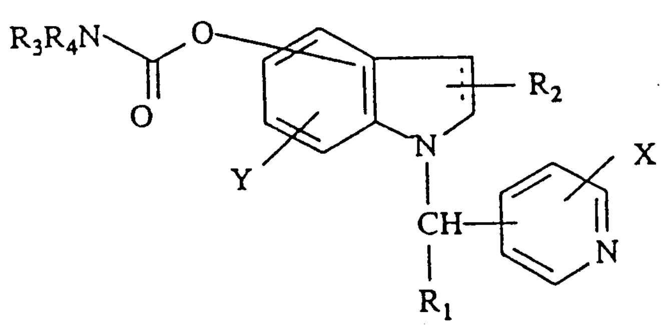

(I)

in welcher

| | |
|---|---|
| $R_1$ | für Wasserstoff, $(C_1$-$C_6)$-Alkyl, Phenyl-$(C_1$-$C_6)$-alkyl, $(C_2$-$C_6)$-Alkenyl oder $(C_2$-$C_6)$-Alkynyl steht; |
| $R_2$ | Wasserstoff, $(C_1$-$C_6)$-Alkyl, $(C_2$-$C_6)$-Alkenyl, Formyl oder Cyano bedeutet; |
| $R_3$ | für Wasserstoff oder $(C_1$-$C_6)$-Alkyl steht; |
| $R_4$ | $(C_1$-$C_6)$-Alkyl, Phenyl-$(C_1$-$C_6)$-alkyl, $(C_3$-$C_8)$-Cycloalkyl, Phenyl, Furanyl, Thienyl, Pyrrolyl, Pyridinyl, Furanyl-$(C_1$-$C_6)$-alkyl, Thienyl-$(C_1$-$C_6)$-alkyl, Pyrrolyl-$(C_1$-$C_6)$-alkyl, Pyridinyl-$(C_1$-$C_6)$-alkyl oder |

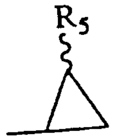

bedeutet, oder $NR_3R_4$ zusammengenommen für

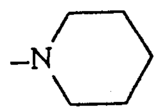 , 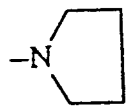 , 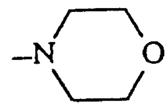

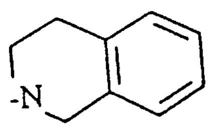 oder 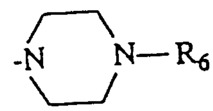

stehen;

$R_5$ Wasserstoff, ($C_1$-$C_6$)-Alkyl oder Phenyl darstellt,

$R_6$ für Wasserstoff, ($C_1$-$C_6$)-Alkyl, Phenyl, Phenyl-($C_1$-$C_6$)-alkyl, Formyl, ($C_1$-$C_6$)-Alkylcarbonyl oder Phenyl-($C_1$-$C_6$)-alkylcarbonyl steht;

X und Y unabhängig voneinander für Wasserstoff, Nitro, Amino, Halogen, ($C_1$-$C_6$)-Alkyl, ($C_1$-$C_6$)-Alkoxy oder Hydroxy stehen,

wobei der Phenylrest bei jedem Auftreten mit einem oder zwei Substituenten substituiert sein kann, die unabhängig voneinander aus ($C_1$-$C_6$)-Alkyl, ($C_1$-$C_6$)-Alkoxy, Halogen oder Trifluormethyl ausgewählt werden, und wobei die gestrichelte Linie eine fakultative Doppelbindung darstellt; oder ihre pharmazeutisch verträglichen Säureadditionssalze und, gegebenenfalls, ihre geometrischen und optischen Isomere und racemischen Gemische.

**2.** Verbindung gemäß Anspruch 1, in welcher $R_3$ für Wasserstoff steht und $R_4$ aus ($C_1$-$C_6$)-Alkyl, Phenyl-($C_1$-$C_6$)-alkyl und ($C_3$-$C_8$)-Cycloalkyl ausgewählt ist und der Phenylrest wie in Anspruch 1 angegeben substituiert sein kann.

**3.** Verbindung gemäß Anspruch 2, in welcher $R_3$ Wasserstoff und $R_4$ ($C_1$-$C_6$)-Alkyl ist.

**4.** Verbindung gemäß Anspruch 2, in welcher $R_3$ Wasserstoff und $R_4$ Phenyl-($C_1$-$C_6$)-alkyl ist, wobei der Phenylrest wie in Anspruch 1 angegeben substituiert sein kann.

**5.** Verbindung gemäß Anspruch 1, die aus den Verbindungen der nachstehenden Gruppe ausgewählt wird:
1-(4-Pyridinylmethyl)-1H-indol-5-ylmethylcarbamat,
1-(4-Pyridinylmethyl)-1H-indol-5-ylbutylcarbamat oder sein Hydrochlorid,
1-(4-Pyridinylmethyl)-1H-indol-5-ylphenylmethylcarbamat,
1-[1-(4-Pyridinyl)butyl]-1H-indol-5-ylmethylcarbamat und
1-[1-(4-Pyridinyl)butyl]-1H-indol-5-ylphenylmethylcarbamat.

**6.** Pharmazeutische Zusammensetzung, die eine Verbindung gemäß Anspruch 1 in einer zur Erleichterung von Gedächtnisstörungen, die durch ein cholinergisches Defizit gekennzeichnet sind, wirksamen Menge und eine pharmazeutisch verträgliche Trägersubstanz dafür enthält.

**7.** Verfahren zur Synthese einer Verbindung gemäß Anspruch 1, in welcher $R_3$ Wasserstoff ist, umfassend das Umsetzen einer Verbindung der Formel (IV)

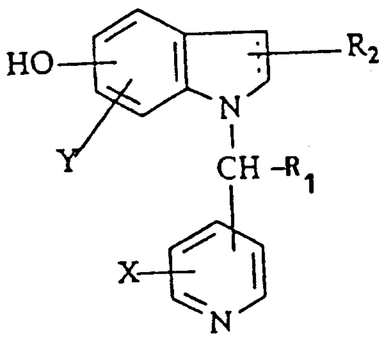

(IV)

mit einem Isocyanat der Formel

$R_4$-N=C=O

in Gegenwart einer Base in einem geeigneten Lösemittel.

**8.** Verfahren gemäß Anspruch 7, wobei das Isocyanat aus Methylisocyanat, Butylisocyanat und Phenylmethylisocyanat ausgewählt wird.

**9.** Verfahren zur Synthese einer Verbindung gemäß Anspruch 1, umfassend das Umsetzen einer Verbindung der Formel (IV)

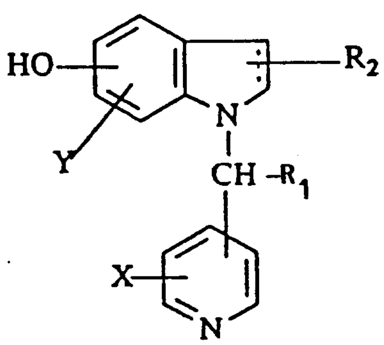

(IV)

mit Carbonyldiimidazol und einem Amin der Formel

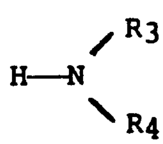

in Gegenwart einer Base in einem geeigneten Lösemittel.

**10.** Verfahren gemäß Anspruch 9, wobei die Base Kaliumcarbonat und das Lösemittel Tetrahydrofuran ist.

**Patentansprüche für folgende Vertragsstaaten : ES, GR**

**1.** Verfahren zur Synthese einer Verbindung der Formel

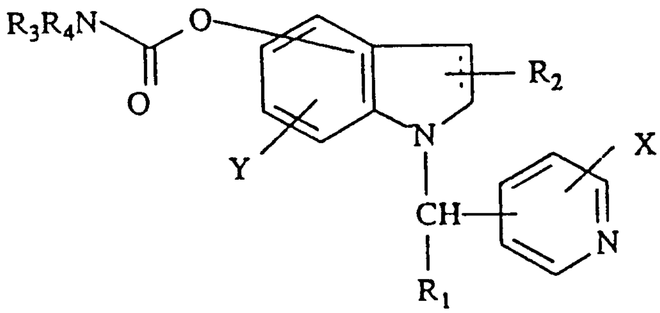

(I)

in welcher

- $R_1$ für Wasserstoff, $(C_1-C_6)$-Alkyl, Phenyl-$(C_1-C_6)$-alkyl, $(C_2-C_6)$-Alkenyl oder $(C_2-C_6)$-Alkynyl steht;
- $R_2$ Wasserstoff, $(C_1-C_6)$-Alkyl, $(C_2-C_6)$-Alkenyl, Formyl oder Cyano bedeutet;
- $R_3$ für Wasserstoff oder $(C_1-C_6)$-Alkyl steht;
- $R_4$ $(C_1-C_6)$-Alkyl, Phenyl-$(C_1-C_6)$-alkyl, $(C_3-C_8)$-Cycloalkyl, Phenyl, Furanyl, Thienyl, Pyrrolyl, Pyridinyl, Furanyl-$(C_1-C_6)$-alkyl, Thienyl-$(C_1-C_6)$-alkyl, Pyrrolyl-$(C_1-C_6)$-alkyl, Pyridinyl-$(C_1-C_6)$-alkyl oder

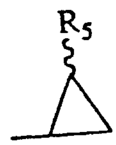

bedeutet, oder $NR_3R_4$ zusammengenommen für

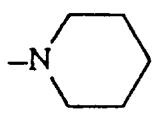
,
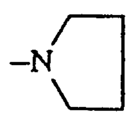
,
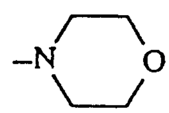
,

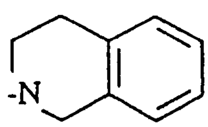
oder
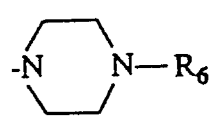

stehen;

- $R_5$ Wasserstoff, $(C_1-C_6)$-Alkyl oder Phenyl darstellt,
- $R_6$ für Wasserstoff, $(C_1-C_6)$-Alkyl, Phenyl, Phenyl-$(C_1-C_6)$-alkyl, Formyl, $(C_1-C_6)$-Alkylcarbonyl oder Phenyl$(C_1-C_6)$-alkylcarbonyl steht;
- X und Y unabhängig voneinander für Wasserstoff, Nitro, Amino, Halogen, $(C_1-C_6)$-Alkyl, $(C_1-C_6)$-Alkoxy oder Hydroxy stehen,

wobei der Phenylrest bei jedem Auftreten mit einem oder zwei Substituenten substituiert sein kann, die unabhängig voneinander aus $(C_1-C_6)$-Alkyl, $(C_1-C_6)$-Alkoxy, Halogen oder Trifluormethyl ausgewählt werden, und wobei die gestrichelte Linie eine fakultative Doppelbindung darstellt;
oder ihrer pharmazeutisch verträglichen Säureadditionssalze und, gegebenenfalls, ihrer geometrischen

und optischen Isomere und racemischen Gemische; umfassend das Umsetzen einer Verbindung der Formel (IV)

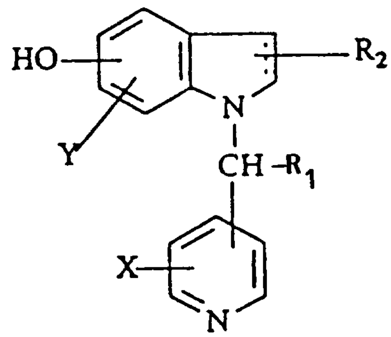

(IV)

mit Carbonyldiimidazol und einem Amin der Formel

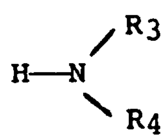

in Gegenwart einer Base in einem geeigneten Lösemittel.

**2.** Verfahren gemäß Anspruch 1, wobei die Base Kaliumcarbonat und das Lösemittel Tetrahydrofuran ist.

**3.** Verfahren zur Synthese einer Verbindung gemäß Anspruch 1, in welcher $R_3$ Wasserstoff ist, umfassend das Umsetzen einer Verbindung der Formel (IV)

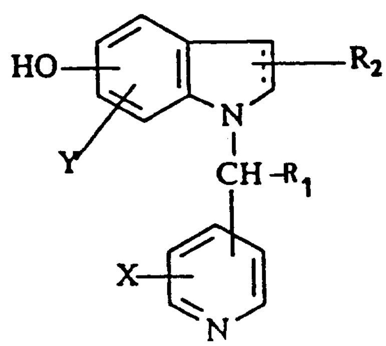

(IV)

mit einem Isocyanat der Formel

$R_4$-N=C=O

in Gegenwart einer Base in einem geeigneten Lösemittel.

**4.** Verfahren gemäß Anspruch 3, wobei das Isocyanat aus Methylisocyanat, Butylisocyanat und Phenylmethylisocyanat ausgewählt wird.

**5.** Verfahren gemäß Anspruch 1 oder 3, zur Herstellung einer Verbindung, die aus den Verbindungen der nachstehenden Gruppe ausgewählt wird:
1-(4-Pyridinylmethyl)-1H-indol-5-ylmethylcarbamat,
1-(4-Pyridinylmethyl)-1H-indol-5-ylbutylcarbamat oder sein Hydrochlorid,
1-(4-Pyridinylmethyl)-1H-indol-5-ylphenylmethylcarbamat,

1-[1-(4-Pyridinyl)butyl]-1H-indol-5-ylmethylcarbamat und
1-[1-(4-Pyridinyl)butyl]-1H-indol-5-ylphenylmethylcarbamat.

## Revendications

### Revendications pour les Etats contractants suivants : AT, BE, CH, DE, DK, FR, GB, IT, LI, LU, NL, SE

**1.** Composé de formule

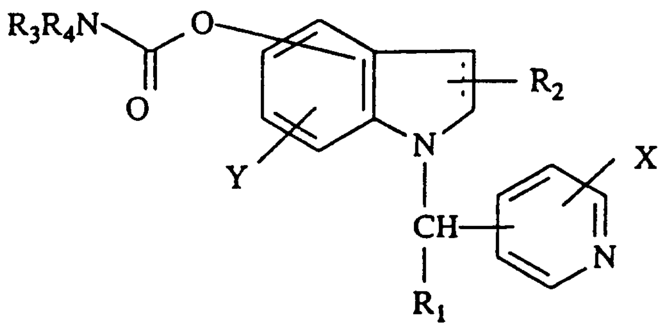

(I)

dans laquelle

- $R_1$ est un atome d'hydrogène ou un groupe alkyle en $C_1$-$C_6$, phényl-alkyle($C_1$-$C_6$), alcényle en $C_2$-$C_6$ ou alcynyle en $C_2$-$C_6$;
- $R_2$ est un atome d'hydrogène ou un groupe alkyle en $C_1$-$C_6$, alcényle en $C_2$-$C_6$, formyle ou cyano;
- $R_3$ est un atome d'hydrogène ou un groupe alkyle en $C_1$-$C_6$;
- $R_4$ est un groupe alkyle en $C_1$-$C_6$, phényl-alkyle($C_1$-$C_6$),cycloalkyle en $C_3$-$C_8$, phényle, furannyle, thiényle, pyrrolyle, pyridinyle, furannyl-alkyle($C_1$-$C_6$), thiényl-alkyle($C_1$-$C_6$), pyrrolyl-alkyle($C_1$-$C_6$), pyridinyl-alkyle($C_1$-$C_6$),

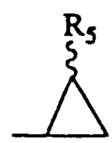 ,

ou $NR_3R_4$ forment ensemble un groupe

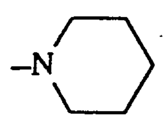 , 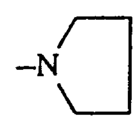 ,

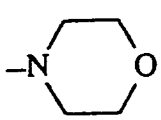 , 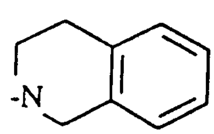 ou 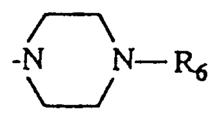 ;

- $R_5$ est un atome d'hydrogène ou un groupe alkyle en $C_1$-$C_6$ ou phényle;
- $R_6$ est un atome d'hydrogène ou un groupe alkyle en $C_1$-$C_6$, phényle, phényl-alkyle($C_1$-$C_6$), formyle, alkyl($C_1$-$C_6$)carbonyle ou phényl-alkyl($C_1$-$C_6$)carbonyle;
- X et Y sont indépendamment un atome d'hydrogène ou d'halogène ou un groupe nitro, amino, alkyle en $C_1$-$C_6$, alcoxy en $C_1$-$C_6$ ou hydroxy,

le radical phényle en chaque occurrence pouvant être substitué par 1 ou 2 subtituants choisis

indépendamment parmi des atomes d'halogène et des groupes alkyle en $C_1$-$C_6$, alcoxy en $C_1$-$C_6$ et trifluorométhyle, et le trait interrompu signifiant une double liaison facultative;
ou sels d'addition avec des acides pharmaceutiquement acceptables de celui-ci et, lorsque cela est applicable, ses isomères géométriques et optiques, et leurs mélanges racémiques.

2. Composé selon la revendication 1, dans lequel $R_3$ est un atome d'hydrogène, et $R_4$ est choisi parmi des groupes alkyle en $C_1$-$C_6$, phényl-alkyle($C_1$-$C_6$) et cycloalkyle en $C_3$-$C_8$, dans lesquels le radical phényle peut être substitué comme indiqué dans la revendication 1.

3. Composé selon la revendication 2, dans lequel $R_3$ est un atome d'hydrogène et $R_4$ est un groupe alkyle en $C_1$-$C_6$.

4. Composé selon la revendication 2, dans lequel $R_3$ est un atome d'hydrogène et $R_4$ est un groupe phényl-alkyle($C_1$-$C_6$), dans lequel le radical phényle peut être substitué comme indiqué dans la revendication 1.

5. Composé selon la revendication 1, qui est choisi parmi
le méthylcarbamate de 1-(4-pyridinylméthyl)-1H-indol-5-yle,
le butylcarbamate de 1-(4-pyridinylméthyl)-1H-indol-5-yle ou son chlorhydrate,
le phénylméthylcarbamate de 1-(4-pyridinylméthyl)-1H-indol-5-yle,
le méthylcarbamate de 1-[1-(4-pyridinyl)butyl]-1H-indol-5-yle et
le phénylméthylcarbamate de 1-[1-(4-pyridinyl)butyl]-1H-indol-5-yle.

6. Composition pharmaceutique comprenant un composé tel que défini dans la revendication 1, en une quantité efficace pour soulager un dysfonctionnement de la mémoire caractérisé par une déficience cholinergique, et un véhicule pharmaceutiquement acceptable pour celui-ci.

7. Procédé de synthèse d'un composé selon la revendication 1, dans lequel $R_3$ est un atome d'hydrogène, comprenant la mise en réaction d'un composé de formule (IV)

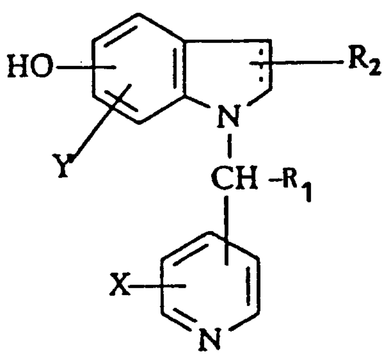

(IV)

avec un isocyanate de formule

$R_4$-N=C=O

en présence d'une base, dans un solvant approprié.

8. Procédé selon la revendication 7, dans lequel l'isocyanate est choisi parmi le méthylisocyanate, le butylisocyanate et le phénylméthylisocyanate.

9. Procédé de synthèse d'un composé selon la revendication 1, comprenant la mise en réaction d'un composé de formule (IV)

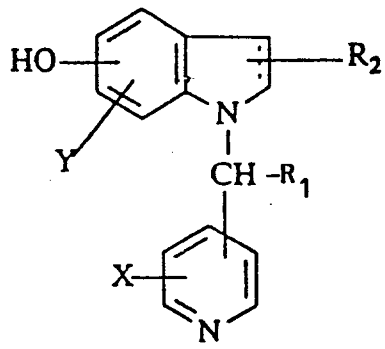

(IV)

avec du carbonyldiimidazole et une amine de formule

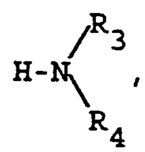

en présence d'une base, dans un solvant approprié.

10. Procédé selon la revendication 9, dans lequel la base est le carbonate de potassium, et le solvant est le tétrahydrofuranne.

**Revendications pour les Etats contractants suivants : ES, GR**

1. Procédé de synthèse d'un composé de formule

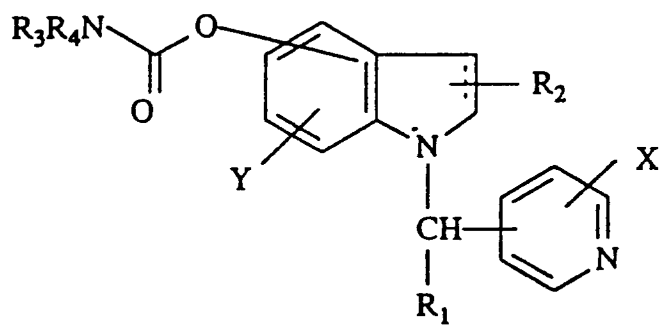

(I)

dans laquelle

- $R_1$ est un atome d'hydrogène ou un groupe alkyle en $C_1$-$C_6$, phényl-alkyle($C_1$-$C_6$), alcényle en $C_2$-$C_6$ ou alcynyle en $C_2$-$C_6$;
- $R_2$ est un atome d'hydrogène ou un groupe alkyle en $C_1$-$C_6$, alcényle en $C_2$-$C_6$, formyle ou cyano;
- $R_3$ est un atome d'hydrogène ou un groupe alkyle en $C_1$-$C_6$;
- $R_4$ est un groupe alkyle en $C_1$-$C_6$, phényl-alkyle($C_1$-$C_6$),cycloalkyle en $C_3$-$C_8$, phényle, furannyle, thiényle, pyrrolyle, pyridinyle, furannyl-alkyle($C_1$-$C_6$), thiényl-alkyle($C_1$-$C_6$), pyrrolyl-alkyle($C_1$-$C_6$), pyridinyl-alkyle($C_1$-$C_6$),

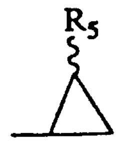 ,

ou $NR_3R_4$ forment ensemble un groupe

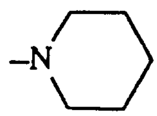 , 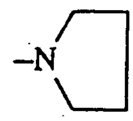 ,

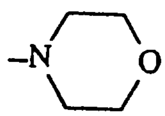 , 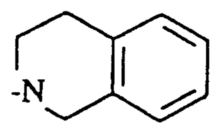 ou 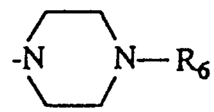 ;

- $R_5$ est un atome d'hydrogène ou un groupe alkyle en $C_1$-$C_6$ ou phényle;
- $R_6$ est un atome d'hydrogène ou un groupe alkyle en $C_1$-$C_6$, phényle, phényl-alkyle($C_1$-$C_6$), formyle, alkyl($C_1$-$C_6$)carbonyle ou phényl-alkyl($C_1$-$C_6$)carbonyle;
- X et Y sont indépendamment un atome d'hydrogène ou d'halogène ou un groupe nitro, amino, alkyle en $C_1$-$C_6$, alcoxy en $C_1$-$C_6$ ou hydroxy,

le radical phényle en chaque occurrence pouvant être substitué par 1 ou 2 subtituants choisis indépendamment parmi des atomes d'halogène et des groupes alkyle en $C_1$-$C_6$, alcoxy en $C_1$-$C_6$ et trifluorométhyle, et le trait interrompu signifiant une double liaison facultative;
ou des sels d'addition avec des acides pharmaceutiquement acceptables de celui-ci et, lorsque cela est applicable, des isomères géométriques et optiques, et leurs mélanges racémiques,
comprenant la mise en réaction d'un composé de formule (IV)

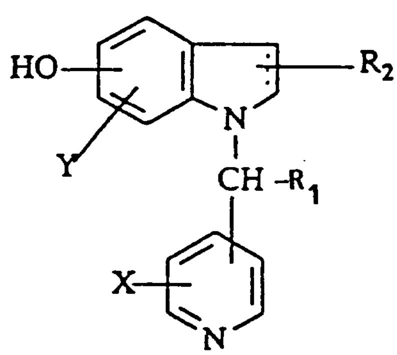

(IV)

avec du carbonyldiimidazole et une amine de formule

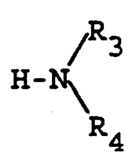 ,

en présence d'une base, dans un solvant approprié.

2. Procédé selon la revendication 1, dans lequel la base est le carbonate de potassium, et le solvant est le tétrahydrofuranne.

3. Procédé de synthèse d'un composé tel que défini à la revendication 1, dans lequel $R_3$ est un atome d'hydrogène, comprenant la mise en réaction d'un composé de formule (IV)

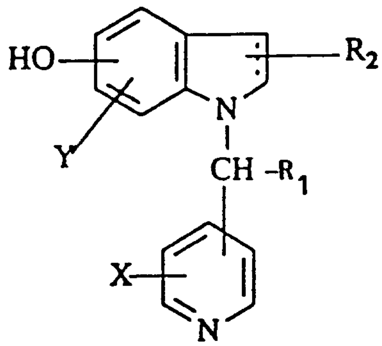

(IV)

avec un isocyanate de formule

$R_4$-N=C=O

en présence d'une base, dans un solvant approprié.

4. Procédé selon la revendication 3, dans lequel l'isocyanate est choisi parmi le méthylisocyanate, le butylisocyanate et le phénylméthylisocyanate.

5. Procédé selon la revendication 1 ou 3, dans lequel on prépare un composé choisi parmi les suivants:
le méthylcarbamate de 1-(4-pyridinylméthyl)-1H-indol-5-yle,
le butylcarbamate de 1-(4-pyridinylméthyl)-1H-indol-5-yle ou son chlorhydrate,
le phénylméthylcarbamate de 1-(4-pyridinylméthyl)-1H-indol-5-yle,
le méthylcarbamate de 1-[1-(4-pyridinyl)butyl]-1H-indol-5-yle et
le phénylméthylcarbamate de 1-[1-(4-pyridinyl)butyl]-1H-indol-5-yle.